# EUROPÄISCHE PATENTANMELDUNG

(11) **EP 2 520 336 A2**
(43) Veröffentlichungstag der Anmeldung: **07.11.2012**
(21) Anmeldenummer: 12166604.4
(22) Anmeldetag: 03.05.2012
(51) Int. Cl.: A61Q 1/08, A61Q 1/12, A61K 8/02, A61K 8/26, A61K 8/25, A61K 8/88, A61K 8/81, A61K 8/44, A61K 8/36, A61K 8/19, A61K 8/87, A61K 8/73, A61K 8/27, A61K 8/29, A61K 8/92, A61K 8/37, A61K 8/893, A61K 8/891, A61K 8/67, A61K 8/31

(54) **Kosmetisches Puderprodukt**

(30) Priorität: 03.05.2011 DE 102011050065
(71) Anmelder: BCM Cosmetic GmbH, 63128 Dietzenbach (DE)
(72) Erfinder: Manderla, Simone, 63128 Dietzenbach (DE)
(74) Vertreter: Meyer-Dulheuer, Karl-Hermann

(57) **Zusammenfassung**

Die vorliegende Erfindung betrifft ein wasserfreies kosmetisches Puderprodukt, welches sowohl feucht als auch trocken aufgetragen werden kann und eine partikuläre Phase aufweist. Die partikuläre Phase enthält mindestens eine hydrophobe pulverförmige Komponente sowie mindestens zwei hydrophile pulverförmige Komponenten. Der Anteil der partikulären Phase beträgt 75 bis 99,90 Gewichts-% und der Anteil an hydrophilen pulverförmigen Komponenten beträgt maximal 14 Gewichts-%, jeweils bezogen auf das Gesamtgewicht der Zubereitung. Der Anteil an hydrophilen pulverförmigen Komponenten umfasst dabei wenigstens einen hydrophilen Filmbildner und einen hydrophilen Glimmer. Wenigstens eine hydrophobe pulverförmige Komponente weist zudem eine hydrophobe Beschichtung auf.

## Beschreibung

Die vorliegende Erfindung betrifft ein wasserfreies kosmetisches Puderprodukt, welches sowohl feucht als auch trocken aufgetragen werden kann und eine partikuläre Phase aufweist. Die partikuläre Phase enthält mindestens eine hydrophobe pulverförmige Komponente sowie mindestens zwei hydrophile pulverförmige Komponenten. Der Anteil der partikulären Phase beträgt 75 bis 99,90 Gewichts-% und der Anteil an hydrophilen pulverförmigen Komponenten beträgt maximal 14 Gewichts-%, jeweils bezogen auf das Gesamtgewicht der Zubereitung. Der Anteil an hydrophilen pulverförmigen Komponenten umfasst dabei wenigstens einen hydrophilen Filmbildner und einen hydrophilen Glimmer. Wenigstens eine hydrophobe pulverförmige Komponente weist zudem eine hydrophobe Beschichtung auf.

Sogenannte "Wet & Dry"-Kompaktpuder-Produkte zeichnen sich dadurch aus, dass diese sowohl feucht unter Zuhilfenahme eines Schwämmchens als auch trocken verwendet werden können. Bei gewöhnlichen Kompaktpuder-Produkten ist dies aufgrund der hydrophilen Eigenschaften der Inhaltsstoffe nicht möglich, da das Wasser in den Puder aufgesaugt wird. Ein gewisser Anteil an hydrophoben Bestandteilen kann ein Aufsaugen des Wassers verhindern und eine Applikation sowohl feucht als auch trocken ermöglichen.

Viele der auf dem Markt erhältlichen Produkte zeigen beim Trocknungsprozess eine deutliche Farbveränderung. Unmittelbar nach dem feuchten Auftragen ist die Farbe dunkel und farbintensiv und ändert sich beim Trocknen in eine hellere und weißliche Farbe. Ebenfalls nachteilig ist ein Spannungsgefühl, welches nach der feuchten Applikation auftritt. In der EP 1 036 555 A1 ist beispielsweise eine kosmetische, hydrophobische, wasserfreie Kompaktpulverzusammensetzung beschrieben, die 20 bis 35% an hydrophilen pulverförmigen Bestandteilen bezogen auf die partikuläre Phase enthält. Hierbei wird auf die Verwendung von beschichteten Bestandteilen weitestgehend verzichtet.

Aufgabe der vorliegenden Erfindung war es, die Nachteile aus dem bekannten Stand der Technik zu überwinden und ein Puderprodukt bereitzustellen, welches sowohl feucht als auch trocken angewendet werden kann. Die Farbeigenschaften des Puderproduktes sollen hierbei sowohl im feuchten als auch im trockenen Zustand stabil bleiben. Weitere Aufgaben und Vorteile der Erfindung ergeben sich aus der nachfolgenden Beschreibung der vorteilhaften Ausgestaltungen und den Beispielen.

Die Aufgabe wird erfindungsgemäß gelöst durch ein wasserfreies kosmetisches Puderprodukt, welches sowohl feucht als auch trocken aufgetragen werden kann und eine partikuläre Phase aufweist. Die partikuläre Phase enthält mindestens eine hydrophobe pulverförmige Komponente sowie mindestens zwei hydrophile pulverförmige Komponenten. Der Anteil der partikulären Phase beträgt 75 bis 99,90 Gewichts-% und der Anteil an hydrophilen pulverförmigen Komponenten beträgt maximal 14 Gewichts-%, jeweils bezogen auf das Gesamtgewicht der Zubereitung. Der Anteil an hydrophilen pulverförmigen Komponenten umfasst dabei wenigstens einen hydrophilen Filmbildner und einen hydrophilen Glimmer. Wenigstens eine hydrophobe pulverförmige Komponente weist zudem eine hydrophobe Beschichtung auf.

Aufgrund des speziellen Verhältnisses zwischen hydrophilen und hydrophoben Bestandteilen und des Zusammenspiels eines hydrophilen Filmbildners mit einem farbstabilisierenden hydrophilen Glimmer ermöglicht es die vorliegende Erfindung, einen Puder bereitzustellen, der sowohl feucht als auch trocken angewendet werden kann, ohne die aus dem Stand der Technik bekannten negativen Produkteigenschaften aufzuweisen.

Als partikuläre Phase wird die Summe aller pulverförmigen Bestandteile im kosmetischen Puderprodukt bezeichnet, wobei die Teilchen vorzugsweise kugelförmig oder plättchenförmig sind. Es werden für Puder übliche Korngrößen verwendet, die vorzugsweise im Bereich zwischen 0,001 µm und 200 µm liegen.

Als ein wasserfreies kosmetisches Puderprodukt im Sinne der Erfindung ist ein Puderprodukt zu verstehen, welches im Wesentlichen frei von Wasser ist. Hierbei beträgt der Wassergehalt des kosmetischen Puderprodukts vorzugsweise unter 2 Gewichts-% bezogen auf das Gesamtgewicht des Puderprodukts.

Das erfindungsgemäße Puderprodukt kann sowohl im trockenen Zustand als auch im feuchten Zustand nach Zugabe von einer geringen Menge Wasser eingesetzt werden. Wenn das Puderprodukt im trockenen Zustand benutzt wird, kann es entweder mit den Fingern oder mit einem Schwamm oder Applikator/Bürste aufgetragen werden. In diesem Falle kann das Produkt als mattierendes Puder eingesetzt werden, welches einen gleichmäßigen Teint verleiht oder als Effekt-Puder, um besondere Farb- oder Glanz-Effekte zu erzielen. Das erfindungsgemäße Puderprodukt kann ebenfalls mittels eines angefeuchteten Schwamms oder Applikators/Bürste aufgetragen werden, um eine erhöhte Deckkraft zu erzielen. Das Puderprodukt kann in diesem Falle wie ein herkömmliches Make-up aufgetragen werden. Nach dem Auftragen auf die Haut behält das erfindungsgemäße Puderprodukt selbst nach der Trocknung einen gleichmäßigen Farbton.

In einer vorteilhaften Ausgestaltungsform der vorliegenden Erfindung beträgt der Anteil an hydrophilen pulverförmigen Komponenten 5 bis 12 Gewichts-%, vorzugsweise 8 bis 10 Gewichts-%, jeweils bezogen auf das Gesamtgewicht der Zubereitung.

In einer weiteren vorteilhaften Ausgestaltung des erfindungsgemäßen Puderprodukts beträgt der Anteil der partikulären Phase 80 bis 90 Gewichts-%, vorzugsweise 85 bis 90 Gewichts-%, jeweils bezogen auf das Gesamtgewicht der Zubereitung.

In einer weiteren bevorzugten Ausgestaltungsform der vorliegenden Erfindung handelt es sich bei mindestens einer der pulverförmigen Komponenten um einen Füllstoff, eine farbgebende Substanz oder Mischungen derselben.

Je nach Bedarf können der Zubereitung Füllstoffe zugesetzt werden, bei denen es sich um farblose oder weiße partikuläre organische oder anorganische Stoffe handelt, die keinen Farbeffekt im Produkt oder auf der Haut hervorrufen. Diese Substanzen sind zudem gekennzeichnet durch keine bis eine geringe Deckkraft. Füllstoffe haben einen positiven Einfluss auf die Textur und können die Produkteigenschaften hinsichtlich Feinheit, Gleichmäßigkeit, Haptik, Haltbarkeit, Natural-Finish und Sebumresistenz verändern. Außerdem können Füllstoffe visuelle Effekte im Endprodukt ermöglichen.

Es ist erfindungsgemäß bevorzugt, wenn der Anteil an Füllstoffen 60 bis 90 Gewichts-%, vorzugsweise 65 bis 80 Gewichts-%, jeweils bezogen auf das Gesamtgewicht der Zubereitung, beträgt.

Als Füllstoffe können anorganische Substanzen, vorzugsweise Talk, Zinkstearate, Kaolin, Bornitrid, Silikate, insbesondere Zeolithe oder Schichtsilikate (Glimmer) eingesetzt werden. Bei den Schichtsilikaten ist die Verwendung von Muskovit, Phlogopit, Biotit, Serizit, Lepidolith, Paragonit, synthetischen Schichtsilikaten oder Mischungen derselben bevorzugt. Zudem können Zinkoxid, Titanoxid, Zirkoniumoxid, Ceroxid, Magnesiumcarbonat und Magnesiumhydrogencarbonat, Calciumcarbonat, Apatit-(CaOH), Glas oder Keramik-Mikrokapseln oder Metallseifen eingesetzt werden. Bei Metallseifen ist die Verwendung von Metallseifen, die von organischen Carbonsäuren mit vorzugsweise 8 bis 22 Kohlenstoff-Atomen abgeleitet sind, bevorzugt (Zink-, Magnesium oder Lithiumstearat, Zinklaurat, Magnesiummyristat und Mischungen derselben). An synthetischen organischen Polymeren können vorzugsweise Polycarbonate, Polyether, Polyester, Polyethylene, Polypropylen, Polyvinylchlorid, Polystyrole, Polyamide, Polyurethane, Polyacrylate, Poly-β-Alanin-Pulver oder Lauroyllysin verwendet werden. Besonders bevorzugt ist die Verwendung von Nylon (Polyamid 6 und Polyamid 12), insbesondere in der Form mikrofeiner Polyamid-Partikel. Weitere bevorzugte organische Füllstoffe können ausgewählt werden aus Polytetrafluorethylen, Polymethylmethacrylaten, Silikonharzen oder Silikonelastomeren, Cellulosen, Stärke oder Stärkederivate.

Bei gefärbten Produkten wie beispielsweise Lidschatten und Kompaktpuder können farbgebende Substanzen eingesetzt werden. Dieses können Farbstoffe, Farbpigmente oder Effektpigmente (wie z.B. Perlglanzpigmente) sein. Diese können sowohl einzeln als auch in einem Gemisch vorliegen sowie gegenseitig miteinander beschichtet sein, um diverse Farbeffekte zu erzielen. Die Partikel können unterschiedlich geformt sein, wie beispielsweise kugelig, oval, plättchenförmig oder ungleichmäßig geformt, sowie in beliebigen Kombinationen der verschiedenen Formen.

Es ist erfindungsgemäß bevorzugt, wenn der Anteil an farbgebenden Substanzen 0,1 bis 20 Gewichts-%, vorzugsweise 0,1 bis 12 Gewichts-%, jeweils bezogen auf das Gesamtgewicht der Zubereitung, beträgt.
- An lipophilen Farbstoffen können beispielsweise Sudan®-Farbstoffe, D&C Red 17, D&C Green 6, Beta-Carotin, D&C Yellow 11, D&C Violet 2, D&C Orange 5, Chinolingelb und Annatto verwendet werden.
- Farbpigmente können aus weißen oder farbigen Pigmenten, anorganischen oder organischen Pigmenten, sowie beschichteten oder unbeschichteten Pigmenten ausgewählt werden. An anorganischen Pigmenten ist die Verwendung von Titandioxid, gegebenenfalls auch beschichtet, Zirkoniumoxid, Zinkoxid, Ceroxid, gelbem, rotem oder schwarzem Eisenoxid, natürlichen Aluminiumsilicaten wie Ocker, Glimmer und Kaolin, manganhaltigen Tonen wie Umbra und roter Bolus, Manganviolett, Ultramarin, Chromoxidgrün, Chromoxidhydratgrün, Chromhydroxid und Berliner Blau bevorzugt. An organischen Pigmenten können Ruß, FD&C Red 40, FD&C Yellow 5, FD&C Blue 1, D&C Green 5, Carmin-Lacke (aus Cochenille), sowie Verlackungen organischer Farbstoffe mit Aluminium, Barium, Calcium, Strontium, Zirkonium oder Mischungen der genannten Stoffe eingesetzt werden.
- Um besondere Farbeffekte zu erzielen, können außerdem verschiedene natürliche Perlglanzpigmente wie beispielweise "Fischsilber" (Guanin/Hypoxanthin-Mischkristalle aus Fischschuppen) oder "Perlmutt" (gemahlene Muschelschalen) eingesetzt werden. Perlglanzpigmente können weiterhin aus den weißen Perlglanzpigmenten ausgewählt werden, wie beispielsweise mit Titandioxid oder mit Bismutchloridoxid (BiOCl) beschichtete Glimmer.
- Außerdem können farbige Effektpigmente wie Titandioxid-Glimmerpigmente mit Eisenoxiden, Titandioxid-Glimmerpigmente insbesondere mit Berliner Blau oder Chromoxid und Titandioxid-Glimmerpigmente mit unterschiedlichen organischen Pigmenten, sowie monokristalline Perlglanzpigmente wie zum Beispiel Bismutchloridoxid eingesetzt werden. Des Weiteren können auch plättchenförmige Metallpulver aus Aluminium, Bronze, Messing, Kupfer, Silber oder Gold verwendet werden. Diese Aufzählungen sind nur beispielhaft und keinesfalls abschließend.

Erfindungsgemäß ist die Verwendung von anorganischen Pigmenten und von Effektpigmenten bevorzugt.

In einer weiteren vorteilhaften Ausführungsform handelt es sich bei der hydrophoben pulverförmigen Komponente im kosmetischen Puderprodukt mindestens um eine der folgenden:
Talk, hydrophobe Polymer-Pulver, vorzugsweise Polyamide, besonders bevorzugt Polyamid 6.6, Polyethylen-Pulver, Polyfluor-Pulver, vorzugsweise Tetrafluorethylen-Polymere, Silikon-Pulver oder Polystyrol-Pulver. Als hydrophobe pulverförmige Komponenten eignen sich zudem Lipo-Aminosäuren, wie beispielsweise Lauryllysin, Bornitride und Metallseifen, die von organischen Carbonsäuren mit vorzugsweise 8 bis 22 Kohlenstoff-Atomen abgeleitet sind (Zink-, Magnesium- oder Lithiumstearat, Zinklaurat, Magnesiummyristat und Mischungen derselben).

Hydrophobe pulverförmige Komponenten können ebenfalls aus hydrophoben oder hydrophilen pulverförmigen Komponenten ausgewählt werden, die hydrophob beschichtet oder behandelt wurden. Die hydrophob beschichteten pulverförmigen Komponenten können vorzugsweise mit einem Silikon beschichtet sein. Alternativ können Metallseifen zur Beschichtung eingesetzt werden, vorzugsweise Magnesiumsterat, Aluminiumstearat oder Zinkstearat. Pulverförmige Komponenten können ebenfalls mit Lecithinen oder pflanzlichen Wachsen, wie Carnaubawachs beschichtet werden. Ebenfalls möglich ist die Verwendung von Aminosäuren, Fluor-Derivaten, Mineralölen, Polyethylen, Polyacrylaten und/oder Mischungen derselben. Bei hydrophob beschichteten und/oder behandelten Bestandteilen ist die Verwendung der folgenden pulverförmigen Komponenten bevorzugt: hydrophob beschichtete und/oder behandelte Glimmer, Silikate, Kaolin, Metalloxide, vorzugsweise Titandioxid, Eisenoxide, Zinkoxide und/oder Mischungen derselben.

In einer alternativen Ausgestaltungsform des kosmetischen Puderprodukts ist wenigstens eine der hydrophoben pulverförmigen Komponenten mit Meticon und/oder Dimeticon beschichtet. Zusätzlich können eine oder mehrere pulverförmige Komponenten mit einem Alkylsilan, vorzugsweise Triethoxycaprylylsilan beschichtet sein.

Besonders bevorzugt ist die Verwendung von beschichtetem Talk, insbesondere beschichtet mit Dimeticon (2%), sowie beschichtet mit einem Alkylsilan, besonders bevorzugt mit Triethoxycaprylylsilan (1%). Die Verwendung von beschichteten Pigmenten der Firma Sensient Cosmetic Technologies ist ebenfalls bevorzugt. Hierbei eignet sich insbesondere mit Triethoxycaprylylsilan (1 %) beschichtetes Titandioxid (Titanium Dioxide AS R3435). Ebenfalls bevorzugt ist die Verwendung von mit Triethoxycaprylylsilan (1 %) beschichteten Eisenoxiden, wie beispielsweise gelbes Eisenoxid, rotes Eisenoxid oder schwarzes Eisenoxid. Unter den Polyamiden ist insbesondere die Verwendung von Nylon-12 bevorzugt, beispielsweise das Produkt Orgasol® 2002 EXD NAT COS der Firma Arkema. Zudem ist die Verwendung von sphärischem Silika (67,5%) gemischt mit Titandioxid (26,5%), welche mit Triethoxycaprylylsilan (6%) beschichtet sind, bevorzugt, beispielsweise als Produkt SH219-AS der Firma Sunjin Chemicals Co., Ltd. Außerdem ist von dieser Firma das Produkt SUNSIL-130HSC bevorzugt, welches ein mit Meticon (3%) beschichtetes Silika ist.

In einer weiteren vorteilhaften Ausgestaltung handelt es sich bei den hydrophilen pulverförmigen Komponenten im kosmetischen Puderprodukt mindestens um eine der folgenden: Phlogopite, Bismutchloridoxid, Silikate, hydrophile Polymere, vorzugsweise Polyacrylate, Polyamide oder Polyurethane, Cellulose- oder Stärkederivate, Kaolin, Apatit-(CaOH), Zinkoxid, Titanoxid, Calciumcarbonat, Magnesiumcarbonat, Magnesiumhydrogen-carbonat und/oder Mischungen derselben.

Besonders bevorzugt für den Einsatz als hydrophiler Glimmer sind die Phlogopite, insbesondere die synthetischen Fluorphlogopite. Das Produkt FNK-100 der Firma Presperse Inc. ist hierbei besonders vorteilhaft. Dieses hat den Effekt, dass selbst eine Zugabe von Feuchtigkeit (bei feuchtem Auftrag des Puderproduktes, oder durch Schwitzen) keine Farbveränderung auf der Haut hervorruft. Das Produkt unterstützt zusätzlich die langanhaltende Farbintensität und wirkt mattierend. Bei den Polyacrylaten ist die Verwendung von Methyl-Methacrylaten besonders bevorzugt. Dieses wirkt als Filmbildner und sorgt für einen gleichmäßigen Film beim Auftragen des Puderprodukts im feuchten Zustand.

Die Kombination dieser beiden Bestandteile ist besonders geeignet, die erfindungsgemäße Wirkung zu erzielen, für die es ganz wesentlich auf die hydrophil/hydrophob-Verteilung der Komponenten und den farbstabilisierenden Effekt der hydrophilen Glimmer ankommt. Ein geringer hydrophiler Pulveranteil stellt eine geringe Wasseraufnahme des Puders sicher und das mit der erfindungsgemäßen Zusammensetzung aufgenommene Wasser wird gezielt zur Filmbildung genutzt, wobei der hydrophile Glimmer eine stabilisierende Wirkung hat. Mit Methyl-Methacrylaten und synthetischen Fluorphlogopiten konnten hierbei besonders gute Ergebnisse erzielt werden.

Pulverförmige Komponenten können zudem mittels verschiedener Materialien hydrophiliert werden. Unter den hydrophil beschichteten und/oder behandelten pulverförmigen Komponenten ist die Verwendung der folgenden bevorzugt: hydrophil beschichtete und/oder behandelte Polyamid-Pulver, Talk, Polyethylen-Pulver, expandierte Vinylidenchlorid-Acetonitril-Methyl(meth)acrylat-Copolymere, Polyfluoro-Pulver, Silikon-Pulver, Polyacrylat-Pulver, Polystyrol-Pulver, Pigmente und Mischungen derselben, hydrophile organische oder anorganische Pigmente.

In einer weiteren vorteilhaften Ausführungsform kann das kosmetisches Puderprodukt zusätzlich mindestens eine Öl-Komponente als Binder aufweisen, wobei der Anteil der Öl-Komponente vorzugsweise 0,1 bis 25 Gewichts-%, besonders bevorzugt 5 bis 13 Gewichts-%, jeweils bezogen auf das Gesamtgewicht der Zubereitung beträgt. Die Öl-Komponenten erleichtern die Adhäsion der pulverförmigen Komponenten an die Haut sowie die Kohäsion der Partikel untereinander in der fertigen Zubereitung.

In einer alternativen Ausgestaltungsform handelt es sich bei der Öl-Komponente um eine der folgenden Substanzen oder die Öl-Komponente enthält eine oder mehrere der folgenden Substanzen:
- Poly(organo)siloxane (Silikone), vorzugsweise Methylcyclopolysiloxan, Diethylpolysiloxan, Methylphenylpolysiloxan, mit Fettsäuren modifiziertes Polysiloxan, mit Aminogruppen modifiziertes Polysiloxan, besonders bevorzugt Methylpolysiloxan und/oder Dimethylpolysiloxan,
- Esteröle, vorzugsweise Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester wie Maisöl, Avocadoöl, Kamelienöl, Olivenöl, Weizenkeimöl, Aprikosenkernöl, Sojaöl, Erdnussöl, Kakaobutter und/ oder Rizinusöl,
- Öle/Wachse, vorzugsweise Öle/tierische Wachse, pflanzliche Öle/Wachse mineralische Öle/Wachse und/oder synthetische Öle/Wachse.

Das erfindungsgemäße kosmetische Puderprodukt kann in einer weiteren Ausführungsform außerdem einen oder mehrere der folgenden Hilfsstoffe enthalten: Spurenelemente, beruhigende Stoffe, oberflächenaktive Substanzen, feuchthaltende Substanzen, rückfettende Agenzien, Fette, Alkohole, Polyole und deren toxikologisch verträglichen Ether und Ester, verzweigte und/oder unverzweigte Kohlenwasserstoffe, Antioxidantien, Vitamine, Emulgatoren, Stabilisatoren, pH-Wert-Regulatoren, Bakterizide, antimikrobielle Stoffe, Maskierungsmittel, Parfums, Silikone, Ceramide, Pflanzenextrakte, Weichmacher, Kohäsionsmittel, Schaumstabilisatoren, Verdickungsmittel oder Konservierungsmittel.

In einer weiteren vorteilhaften Ausgestaltungsform handelt es sich beim kosmetischen Puderprodukt um einen Puder, einen Körperpuder, ein Rouge, einen Lidschatten oder einen Augenbrauenpuder.

Bei dem erfindungsgemäßen kosmetischen Puderprodukt kann es sich sowohl um ein gepresstes als auch um ein loses Puderprodukt handeln.

Die Erfindung soll nachstehend durch Beispiele erläutert werden, welches sie jedoch nicht abschließend beschreibt. Die Mengenangaben erfolgen hierbei in Gewichtsprozent, jeweils bezogen auf das Gesamtgewicht der Zubereitung. Zur Kennzeichnung der Rohstoffe werden die dem einschlägigen Fachmann bekannten INCI-Namen verwendet.

**Tabelle 1: Zusammensetzung für kosmetisches Puderprodukt**

| ***Rohstoff*** | ***Menge*** |
|---|---|
| Talk - hydrophob beschichtet | 38,350 |
| Mica - hydrophob beschichtet | 15,000 |
| Pigmente - hydrophob beschichtet | 11,500 |
| Synthetisches Fluorphlogopit | 8,750 |
| weitere Füllstoffe | 15,000 |
| Methyl Methacrylat Crosspolymer | 0,500 |
| Öle | 10,000 |
| Hilfsstoffe | 0,900 |

In dieser Zusammensetzung sind ein synthetisches Fluorphlogopit und ein Methyl Methacrylat Crosspolymer als hydrophile pulverförmige Komponenten eingesetzt, die zusammen 9,25 Gewichts-%, bezogen auf das Gesamtgewicht der Zubereitung ausmachen. Der Anteil der partikulären Phase beträgt in diesem Ausführungsbeispiel 90,00 Gewichts-%, bezogen auf das Gesamtgewicht der Zubereitung.

Die Eigenschaften eines erfindungsgemäßen "Wet & Dry"-Kompaktpuder-Produkts wurde in einem nächsten Schritt mit einem "Wet & Dry"-Produkt aus dem Stand der Technik verglichen. Die Zusammensetzungen wurden auf Basis der INCI-Zusammensetzungen (Pigmente nicht angegeben) miteinander verglichen.

**Tabelle 2: Vergleich von "Wet & Dry"-Kompaktpuder-Produkten**

| ***Erfindungsgemäße Zubereitung*** | ***Zubereitung Stand der Technik*** |
|---|---|
| Talc | Talc |
| Mica | Mica |
| Nylon-12 | Aluminum Starch Ocetenylsuccinate |
| Synthetic Fluorphlogopite | |
| Silica | |
| Isopropyl Isostearate | |
| Dimethicone | Dimethicone |
| Squalane | Magnesium Myristate |
| Bis-Hydroxyethoxypropyl Dimethicone | |
| Trimethylsiloxysilicate | Trimethylsiloxysilicate |
| Triethoxycaprylysilane | Boron Nitride |
| Apricot Kernel Oil Polyglyceryl-4 Esters | Phenyl Trimethicone |
| Apricot Kernel Oil Polyglyceryl-3 Esters | Dimethiconol |
| Methyl Metacrylate Crosspolymer | Cyclomethicone |
| Pentaerythrityl Tetraisostearate | |
| Apricot Kernel Oil Polyglyceryl-5 Esters | |
| Methylparaben | |
| Tocopherol Acetate | Tocopherol Acetate |
| Apricot Kernel Oil Polyglyceryl-6 Esters | Phenoxyethanol |
| | Methylparaben |
| | Butylparaben |
| | Ethylparaben |
| Propylparaben | Propylparaben |
| Methicone | Isobutylparaben |

Die beiden Kompaktpuder-Produkte wurden sowohl im feuchten als auch im trockenen Zustand auf die Gesichtshaut aufgetragen. Verglichen wurde zunächst die Abgabe an Partikeln von der Kompaktpuder-Zubereitung. In einem weiteren Schritt wurde untersucht, wie gut sich die beiden Produkte im feuchten beziehungsweise im trockenen Zustand auftragen lassen. Besonders wichtig war, ob nach der Trocknung des feucht aufgetragenen Kompaktpuders eine Farbveränderung auf der Haut erfolgte oder ob der Farbeindruck gleichbleibend war.

Es zeigt sich, dass das erfindungsgemäße Produkt im Vergleich zum Stand der Technik eine gute Abgabe sowohl im feuchten als auch im trockenen Zustand aufweist. Das Vergleichsprodukt hingegen weist eine sehr harte Konsistenz auf und das Produkt gibt kaum Puder-Partikel ab, so dass nach dem Auftrag auf die Haut fast kein Effekt zu sehen ist. Zudem ist das Vergleichsprodukt sehr inhomogen. Das erfindungsgemäße Produkt lässt sich problemlos sowohl feucht als auch trocken auftragen und führt nach Trocknung zu keiner Farbveränderung auf der Haut. Das Vergleichsprodukt hat jedoch den Nachteil, dass es nach der Trocknung auf der Haut eine weißliche Farbe annimmt, welche nicht gewünscht ist und zu einem unschönen Effekt auf der Haut führt.

## Patentansprüche

1. Wasserfreies kosmetisches Puderprodukt, welches sowohl feucht als auch trocken aufgetragen werden kann und eine partikuläre Phase aufweist, wobei die partikuläre Phase mindestens folgende Komponenten enthält:
- mindestens eine hydrophobe pulverförmige Komponente,
- mindestens zwei hydrophile pulverförmige Komponenten,
**dadurch gekennzeichnet, dass**
- der Anteil der partikulären Phase 75 bis 99,90 Gewichts-% beträgt und
- der Anteil an hydrophilen pulverförmigen Komponenten maximal 14 Gewichts-%, jeweils bezogen auf das Gesamtgewicht der Zubereitung, beträgt und wenigstens einen hydrophilen Filmbildner und einen hydrophilen Glimmer umfasst und
- wenigstens eine hydrophobe pulverförmige Komponente eine hydrophobe Beschichtung aufweist.

2. Kosmetisches Puderprodukt nach Anspruch 1, **dadurch gekennzeichnet, dass** der Anteil an hydrophilen pulverförmigen Komponenten 5 bis 12 Gewichts-%, vorzugsweise 8 bis 10 Gewichts-%, jeweils bezogen auf das Gesamtgewicht der Zubereitung, beträgt.

3. Kosmetisches Puderprodukt nach einem der vorherigen Ansprüche, **dadurch gekennzeichnet, dass** der Anteil der partikulären Phase 80 bis 90 Gewichts-%, vorzugsweise 85 bis 90 Gewichts-%, jeweils bezogen auf das Gesamtgewicht der Zubereitung, beträgt.

4. Kosmetisches Puderprodukt nach einem der vorherigen Ansprüche, **dadurch gekennzeichnet, dass** es sich bei mindestens einer der pulverförmigen Komponenten um einen Füllstoff, eine farbgebende Substanz oder Mischungen derselben handelt.

5. Kosmetisches Puderprodukt nach einem der vorherigen Ansprüche, **dadurch gekennzeichnet, dass** es sich bei der hydrophoben pulverförmigen Komponente mindestens um eine der folgenden handelt:
Talk, hydrophobe Polymer-Pulver, vorzugsweise Polyamide, besonders bevorzugt Polyamid 6.6, Polyethylen-Pulver, Polyfluor-Pulver, vorzugsweise Tetrafluorethylen-Polymere, Silikon-Pulver, Polystyrol-Pulver, Lipo-Aminosäuren, vorzugsweise Lauroyllysin, Bornitride, Metallseifen, die von organischen Carbonsäuren mit vorzugsweise 8 bis 22 Kohlenstoff-Atomen abgeleitet sind, hydrophob beschichtete und/oder behandelte pulverförmige Komponenten und/oder Mischungen derselben.

6. Kosmetisches Puderprodukt nach einem der vorherigen Ansprüche, **dadurch gekennzeichnet, dass** wenigstens eine der hydrophoben pulverförmigen Komponenten mit wenigstens einer der folgenden Substanzen beschichtet ist:
einem Silikon, vorzugsweise Meticon und/oder Dimeticon, Metallseifen, vorzugsweise Magnesiumsterat, Aluminiumstearat oder Zinkstearat, Lecithinen, pflanzlichen Wachsen, vorzugsweise Carnaubawachs, Aminosäuren, Fluor-Derivate, Mineralöle, Polyethylen, Polyacrylaten und/oder Mischungen derselben.

7. Kosmetisches Puderprodukt nach einem der vorherigen Ansprüche, **dadurch gekennzeichnet, dass** es sich bei den hydrophilen pulverförmigen Komponenten mindestens um eine der folgenden handelt:
Phlogopite, Bismutchloridoxid, Silikate, hydrophile Polymere, vorzugsweise Polyacrylate, Polyamide oder Polyurethane, Cellulose- oder Stärkederivate, Kaolin, Apatit-(CaOH), Zinkoxid, Titanoxid, Calciumcarbonat, Magnesiumcarbonat, Magnesiumhydrogencarbonat, hydrophil beschichtete und/oder behandelten pulverförmigen Komponenten und/oder Mischungen derselben.

8. Kosmetisches Puderprodukt nach einem der vorherigen Ansprüche, **dadurch gekennzeichnet, dass** es zusätzlich mindestens eine Öl-Komponente aufweist, wobei der Anteil der Öl-Komponente vorzugsweise 0,1 bis 25 Gewichts-%, besonders bevorzugt 5 bis 13 Gewichts-%, jeweils bezogen auf das Gesamtgewicht der Zubereitung beträgt.

9. Kosmetisches Puderprodukt nach einem der vorangehenden Ansprüche, **dadurch gekennzeichnet, dass** es sich bei der Öl-Komponente um eine der folgenden Substanzen handelt oder dass die Öl-Komponente eine oder mehrere der folgenden Substanzen enthält:
- Poly(organo)siloxane (Silikone), vorzugsweise Methylcyclopolysiloxan, Diethylpolysiloxan, Methylphenylpolysiloxan, mit Fettsäuren modifiziertes Polysiloxan, mit Aminogruppen modifiziertes Polysiloxan, besonders bevorzugt Methylpolysiloxan und/oder Dimethylpolysiloxan,
- Esteröle, vorzugsweise Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester wie Maisöl, Avocadoöl, Kamelienöl, Olivenöl, Weizenkeimöl, Aprikosenkernöl, Sojaöl, Erdnussöl, Kakaobutter und/ oder Rizinusöl,
- Öle/Wachse, vorzugsweise tierische Öle/Wachse, pflanzliche Öle/Wachse mineralische Öle/Wachse und/oder synthetische Öle/Wachse.

10. Kosmetisches Puderprodukt nach einem der vorangehenden Ansprüche, **dadurch gekennzeichnet, dass** das kosmetischen Puderprodukt einen oder mehrere der folgenden Hilfsstoffe enthält: Spurenelemente, beruhigende Stoffe, oberflächenaktive Substanzen, feuchthaltende Substanzen, rückfettende Agenzien, Fette, Alkohole, Polyole und deren toxikologisch verträglichen Ether und Ester, verzweigte und/oder unverzweigte Kohlenwasserstoffe, Antioxidantien, Vitamine, Emulgatoren, Stabilisatoren, pH-Wert-Regulatoren, Bakterizide, antimikrobielle Stoffe, Maskierungsmittel, Parfums, Silikone, Ceramide, Pflanzenextrakte, Weichmacher, Kohäsionsmittel, Schaumstabilisatoren, Verdickungsmittel oder Konservierungsmittel.

11. Kosmetisches Puderprodukt nach einem der vorangehenden Ansprüche, **dadurch gekennzeichnet, dass** es sich beim kosmetischen Puderprodukt um einen Puder, einen Körperpuder, ein Rouge, einen Lidschatten oder einen Augenbrauenpuder handelt.
